# EUROPEAN PATENT SPECIFICATION

(11) **EP 4 403 917 B1**
(45) Date of publication and mention of the grant of the patent: **16.07.2025**
(21) Application number: 23382051.3
(22) Date of filing: 23.01.2023
(51) Int. Cl.: G01N 33/543, G01N 35/00

(54) **READING METHOD OF A LATERAL FLOW TEST**
LESEVERFAHREN EINES LATERALFLUSSTESTS
PROCÉDÉ DE LECTURE D'UN TEST DE FLUX LATÉRAL

(43) Date of publication of application: 24.07.2024
(73) Proprietor: P4Q Health, S.L., 48810 Alonsotegi (ES)
(72) Inventor: VEGAS DIEZ, David, 39880 Valle de Villaverde (ES); KOBEAGA URRIOLABEITIA, Gorka, 48300 GERNIKA-LUMO (ES)
(74) Representative: Igartua, Ismael

(56) References cited:
- US-A1- 2013 273 528
- US-A1- 2015 160 134
- US-A1- 2021 364 536

## Description

### TECHNICAL FIELD

The present invention relates to *in vitro* diagnostic testing by means of lateral flow used to detect the presence or absence of an analyte of interest in a sample of biological fluid from a patient.

### PRIOR ART

Lateral flow immunoassays, or lateral flow tests (LFT), are medical tests used to detect the presence or absence of an analyte of interest in a sample of biological fluid obtained from a patient. The lateral flow test comprise a cassette that has a window through which a test strip arranged inside the cassette is observed, the test strip having one or more test lines and one control line. The cassette has a well where the sample to be analyzed is deposited, after which the sample is diffused by capillarity along the test strip, and once a reaction time has elapsed (which can range between 3 and 30 minutes) the results in the test strip are obtained and can be viewed through the window of the cassette. When the sample from the patient contains the analyte of interest, the test line is colored in the test strip, with the test being positive, and if the sample does not contain the analyte, the test line is not colored, with the test being negative. The control line must always be colored in the test strip after depositing the sample, otherwise the test is not valid.

The results of a lateral flow test can be read with the naked eye, or they can be read by means of a reader that performs an automatic reading. The reader usually has optical means, such as a light emitter that illuminates the test strip and a photodiode that receives the light reflected by the test strip to generate a result, such as "positive" or "negative". Lateral flow tests can be used for various types of testing, such as pregnancy detection, detection of antigens indicating virus infection, detection of disease biomarkers, metabolites and other molecular targets, as well as detection of animal diseases, chemicals, toxins, water pollutants, among others.

In laboratories it is common to use readers that perform accurate automatic readings, such as the readers described in WO2018031786A1 or US20190086431A1. These readers have a motor for controlled introduction of the lateral flow test into a reader housing where an optical unit with a light emitter and a sensor is arranged to obtain an accurate reading of the test, and the result is reproduced on a digital display. Although these motorized readers allow accurate readings to be obtained, they are expensive equipment and the patient generally has to wait approximately 24 to 48 hours for the sample to be sent to the laboratory and processed for the result to be known.

The worldwide pandemic caused by the SARS-CoV-2 virus has popularized the use of lateral flow tests for self-diagnosis and has generated the need for tests on the market that allow results to be obtained in the shortest possible time and with the highest possible accuracy rate. In this regard, US20210364536A1 shows a reader for a lateral flow test that can be used directly by a patient and that does not require a motor to introduce the test, but rather the test is introduced manually.

Namely, US20210364536A1 shows a reading method of a lateral flow test that uses a cassette and a reader that has a housing for receiving the cassette. The cassette has a window through which a test strip arranged inside the cassette is observed, the test strip having a reactive zone with test lines and one control line. The reader has an optical unit with a light emitter for illuminating the cassette and a photodiode for receiving the light reflected by the cassette. The cassette is manually moved in the housing of the reader and an output signal comprising luminous intensity values of the light reflected by the cassette during the time that the cassette moves is obtained with the sensor. After obtaining the output signal, the luminous intensity values of the output signal are normalized in a range defined between a lower value and a higher value, then peaks in the output signal corresponding with the test and control lines are identified, and lastly the luminous intensity of the peaks is quantified. The concentration of the analyte of interest is related to the light reflected in the test line, and the higher the concentration is, the greater the intensity (color) of the test line is.

The reader has a tray in which is arranged the cassette with the test strip, and the tray is introduced inside the housing of the reader in which the light emitter and the photodiode are located. The tray has a calibration pattern with several parallel lines the darkness of which gradually varies from the lower value to the higher value, for example, on a gray scale. Every time a reading is performed, first, the photodiode receives the light reflected by the lines of the calibration pattern, and then it receives the light reflected by the test strip. In this way, every time a test strip reading is performed, the luminous intensity values of the light reflected by the test strip are normalized with the luminous intensity values of the light reflected by the lines of the calibration pattern which is arranged on the tray. In this sense, the readings of the photodiode are corrected due to non-linearity in the optics or electronics of the reader. In other words, the coordinates of the Y axis of the signal obtained with the photodiode are normalized with the calibration pattern.

In addition, the reader has an optical position encoder in the tray which is used to relate the time readings obtained by the photodiode to linear positions of the tray. In this way, each luminous intensity value of the light reflected by the test strip corresponds to a known linear position of the test strip. Thus, the reader can recognize the linear positions of the photodiode signal where the peaks corresponding to the test and control lines are expected to be, and therefore, the photodiode readings are independent of the speed at which the user removes the tray from the reader. In other words, with the optical encoder the X axis coordinates of the photodiode output signal are normalized.

### DISCLOSURE OF THE INVENTION

The object of the invention is to provide a reading method of a lateral flow test, as defined in the claims.

The invention relates to a reading method of a lateral flow test comprising:
- using a cassette that has a window through which a test strip arranged inside the cassette is observed, the test strip having a reactive zone with at least one test line and one control line,
- using a reader that has a housing for receiving the cassette, the reader having an optical unit with a light emitter for illuminating the cassette and a sensor for receiving the light reflected, or emitted, by the cassette,
- manually moving the cassette in the housing of the reader,
- obtaining with the sensor an output signal comprising luminous intensity values of the light reflected, or emitted, by the cassette during the time that the cassette moves,
- normalizing the luminous intensity values of the output signal of the sensor in a range defined between a lower value and a higher value, and
- identifying peaks in the output signal of the sensor, said peaks corresponding with the test line and the control line.

In the normalization, the lower value and the higher value are established between the light reflected, or emitted, by the cassette outside the window, and the light reflected, or emitted, by the test strip outside the reactive zone. The lower value corresponds with the light reflected, or emitted, by the cassette outside the window, and the higher value corresponds with the light reflected, or emitted, by the test strip outside the reactive zone. The cassette has a different color than the test strip which causes the luminous intensity of the light reflected, or emitted, by the cassette outside the window to be different from the luminous intensity of the light reflected, or emitted, by the test strip outside the reactive zone.

In this way, only information about the light reflected, or emitted, by the cassette that has the test strip is used to normalize the luminous intensity values of the output signal of the sensor. In other words, the method does not require using an additional calibration pattern to correct possible failures in the output signal of the sensor, for example, failures due to non-linearity in the optics or electronics of the reader. For example, US20210364536A1 uses a tray to place the cassette that has the test strip, and said tray has a known calibration pattern with several parallel lines on a gray scale. Every time the test strip of a cassette is read, the calibration pattern of the tray must be read first to normalize the luminous intensity values of the test strip reading, establishing a relationship between the calibration pattern reading and the test strip reading. The method proposed by the invention does not require using a tray with a calibration pattern for normalizing, since it uses known information about the light reflected, or emitted, by the cassette itself inside and outside the window where the test strip is located. The cassette outside the window and the test strip outside the reactive zone are known, and therefore information about the light reflected in those zones is used to establish the upper and lower values with which the output signal of the sensor is normalized. All the luminous intensity values of the light reflected, or emitted, by the cassette with the test strip are established proportionally according to said upper and lower values.

These and other advantages and feature of the invention will become apparent in view of the figures and detailed description of the invention.

### DESCRIPTION OF THE DRAWINGS

Figure 1a shows an example of a lateral flow test which is manually introduced into a reader such as the one shown in Figure 1b.
Figure 2 shows a diagram of the membranes making up a test strip of a lateral flow test.
Figure 3 shows a flow chart of an example of the reading method of a lateral flow test.
Figure 4a shows raw data of the output signal of a sensor in a single wavelength and Figure 4b shows the signal of Figure 4a after normalization.
Figure 5a shows raw data of the output signal of a spectrometric sensor in several wavelengths and Figure 5b shows the signal of Figure 5a after normalization.

### DETAILED DISCLOSURE OF THE INVENTION

The invention relates to a reading method of a lateral flow test 10 which is used to detect the presence or the absence of an analyte in a liquid sample from a patient. The lateral flow test 10 is read automatically by means of a reader 20.

The lateral flow test may be used in different biological testing scenarios; by way of nonlimiting example, they may be used for the detection of bacteria or viruses, pregnancy or fertility, heart infections, diabetes, the detection of cancer biomarkers, or therapeutic drug monitoring (TDM).

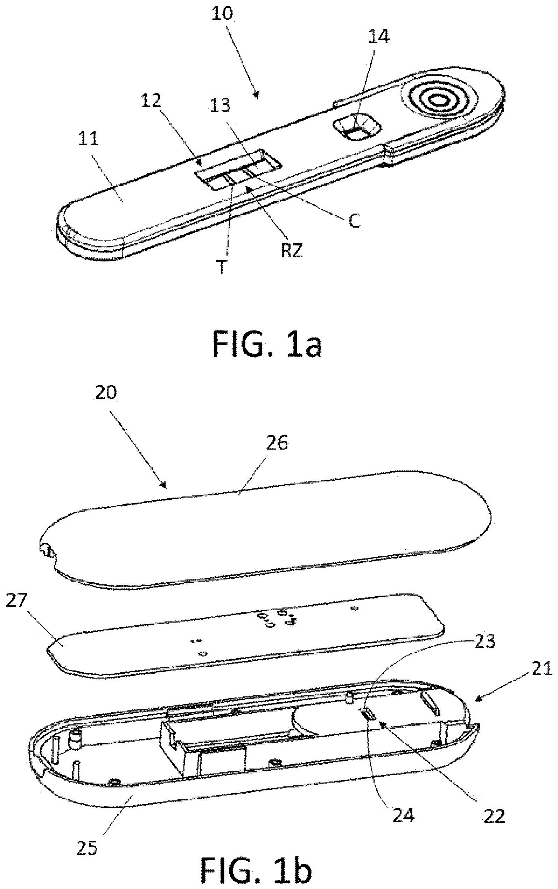

The lateral flow test 10 comprises a cassette 11 that has a window 12 through which a test strip 13 arranged inside the cassette 11 is observed. The test strip 13 has a reactive zone RZ with at least one test line T and one control line C. The cassette 11 has a well 14 in which the liquid sample from the patient is deposited. See Figure 1a.

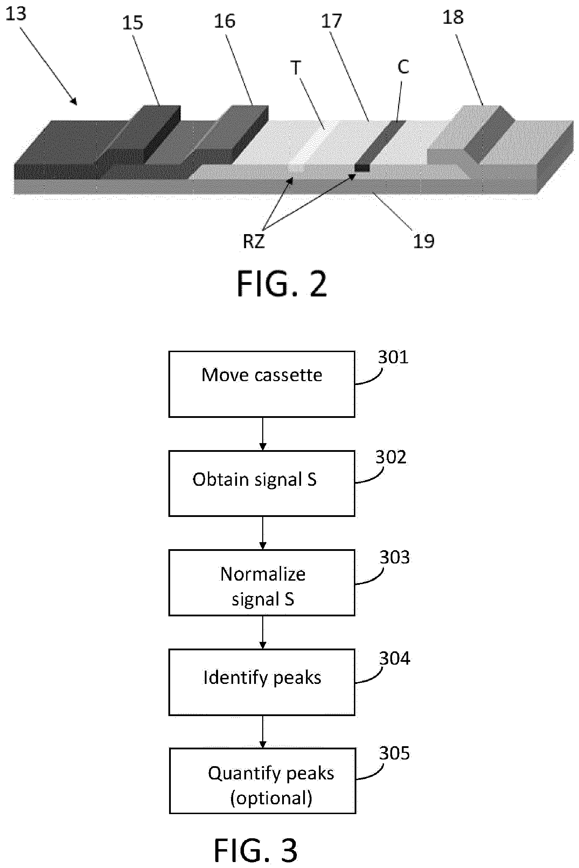

The test strip 13 comprises membranes 15, 16, 17 and 18 that are superimposed on one another and adhered on a lower support 19. The membranes allow the liquid sample from the patient to diffuse by capillarity. The membranes comprise a sample pad 15, a conjugate pad 16, a porous membrane 17, and an absorbent pad 18. See Figure 2.

The liquid sample is applied in the well 14 which is directly communicated with the sample pad 15, which ensures that the analyte present in the sample is capable of binding to the capture reagents for capturing the conjugates arranged in the porous membrane 17. The treated sample migrates through the conjugate pad 16, which contains antibodies specific to the target analyte and conjugate with colored or fluorescent particles, (commonly latex or colloidal gold microspheres). The sample, together with the conjugated antibody bound to the target analyte, migrates to the porous membrane 17 (normally made up of nitrocellulose) with specific biological components (mainly antibodies or antigens) immobilized on a test line T which is oriented perpendicular to the longitudinal axis of the test strip 13. Their function is to react with the analyte bound to the conjugated antibody. Recognition of the analyte of the sample results in a coloring of the test line T, whereas a response in the control line C indicates that a suitable amount of liquid flow has passed through the entire test strip 13. Finally, the absorbent pad 18 absorbs the excess reagents and prevents reflux of the liquid.

The reading, represented by the coloring of the test line T and control line C, which are colored with different intensities, can be evaluated with the naked eye or by using the reader 20. In some cases an ultraviolet light is required to visualize the coloring of the lines. The reader 20 can correlate the color intensity of the test line T with the concentration of the analyte in the liquid sample from the patient.

The reader 20 has a housing 21 for receiving the cassette 11. The reader 20 has an optical unit 22 with a light emitter 23 for illuminating the cassette 11 and a sensor 24 for receiving the light reflected, or emitted, by the cassette 11. See Figure 1b.

The cassette 11 may fit snugly moved in the housing 21 of the reader 20, and the housing 21 may be opaque so that the reading of the sensor 24 is not affected by outside light. The reader 20 comprises a base 25 that is closed in the upper part with a cover 26, and the housing 21 for receiving the cassette 11 is defined between the base 25 and the cover 26. An electronics board 27 is also arranged between the base 25 and the cover 26.

According to one embodiment, the light emitter 23 emits visible light on the cassette 11 (including the test strip 13), and the sensor 24 receives the light directly reflected by the cassette 11. In this way, the reader 20 may be used with a lateral flow test which works according to the principle of reflectance (colorimetry). When visible light is applied on the control and test lines, the light reflected is reduced due to the particles present in the lines that absorb light at a certain wavelength. The particles can be colloidal gold particles or particles of another type initially present in the conjugate pad 16, such as latex particles, carbon nanoparticles, etc.

According to another embodiment, the light emitter 23 emits UV light which is absorbed by the cassette 11 (including the test strip 13), and the sensor 24 receives visible light emitted by the cassette 11, as a contrast to the UV light. In this way, the reader 20 may be used with a lateral flow test which works according to the principle of luminescence. The control line C and test line T of the test strip 13 contain fluorescent particles which, upon receiving UV light, absorb UV light and in turn emit a visible light which is captured by the sensor 24.

The reading method of the lateral flow test 10 comprises using the cassette 11 that has the test strip 13 with the test line T and the control line C and using the reader 20 that has the optical unit 22 with the light emitter 23 for illuminating the cassette 11 and the sensor 24 for receiving the light reflected, or emitted, by the cassette 11.

Figure 3 shows a flow chart of the steps of the reading method. In a first step 301, after obtaining the sample from the patient and depositing it in the well 14 of the cassette 11, the cassette 11 is manually moved in the housing 21 of the reader 20. The light emitter 23 of the optical unit 22 sends light which illuminates the cassette 11 and the test strip 13 during the movement of the cassette 11, and the sensor 24 of the optical unit 22 receives the light reflected, or emitted, by the cassette 11 and the test strip 13. The light reflected, or emitted, by the cassette 11 and the test strip 13 is related to the light sent by the light emitter 23.

In a second step 302, an output signal S comprising luminous intensity values of the light reflected by the cassette 11 during the time that the cassette 11 moves is obtained with the sensor 24. The output of the sensor 24 represents raw data from the reading of the sensor 24. The output signal S represents luminous intensity values "Iv" measured by the sensor 24 during the time "t" that the cassette 11 is manually moved. The Y axis of the signal shows luminous intensity values "Iv" and the X axis shows time "t".

The cassette 11 is manually moved between a first position in which the window 12 of the cassette 11 is inside the housing 21 of the reader 20 and a second position in which the window 12 of the cassette 11 is outside the housing 21 of the reader 20. Preferably, the signal S is obtained during the movement of the cassette 11 between the first and the second position, i.e., during the removal of the cassette 11 from the housing. Alternatively, the signal S may be obtained during the movement of the cassette 11 between the second and the first position, i.e., during the introduction of the cassette 11 into the housing. Alternatively, two signals may be obtained, one during the introduction and the other during the removal, with one of the signals being used as a redundant signal.

In a third step 303, the luminous intensity values of the output signal S are normalized in a range R defined between a lower value and a higher value. The raw readings of the sensor 24 are normalized in the range R defined between said lower value and higher value so that all the readings performed with the sensor 24 are represented on a common scale and are comparable with other readings. For example, normalization allows the correction of possible failures due to non-linearity in the optics or electronics of the reader. The Y axis of the signal shows adimensional values in the range R and the X axis shows time "t". The X coordinates of the output signal S may be discrete readings of the sensor captured every 9 ms.

In a fourth step 304, peaks in the output signal S corresponding with the test line T and the control line C are identified. The identification of the peaks allows detection of the presence of an analyte in the test line T, and therefore detection of the test being 'positive', and also allows detection of the presence of the control line, and therefore detection of the test being 'valid'.

In an optional fifth step 305, after identifying the peaks in the output signal S, the luminous intensity of the peak of the test line T is quantified, with the luminous intensity being related to the concentration of an analyte present in the test line T. Generally, the control line C usually exhibits a similar luminous intensity when the test is valid, but the test line T usually exhibits a different luminous intensity depending on the concentration of the analyte present in the test line T.

According to the invention, in the normalization of the third step 303, the lower value and the higher value are established between the light reflected, or emitted, by the cassette 11 outside the window 12, and the light reflected, or emitted, by the test strip 13 outside the reactive zone RZ. Preferably, in the normalization of the third step 303, the lower value corresponds with the light reflected, or emitted, by the cassette 11 outside the window 12, and the higher value corresponds with the light reflected, or emitted, by the test strip 13 outside the reactive zone RZ.

Thus, the luminous intensity values of the output signal S of the sensor 24 are normalized (scaled) in the range R defined between said lower value and said higher value. All the luminous intensity values of the light reflected, or emitted, by the cassette 11 and the test strip 13 are established proportionally according to said upper and lower values.

The cassette 11 is a known element, since the color and the type of material used for manufacturing same are known, so the luminous intensity value of the light reflected by the cassette 11 outside the window 12 is known. Moreover, the test strip 13 is also a known element, since its color (white) and the material used for manufacturing same are known, so the luminous intensity value of the light reflected by the test strip 13 is also known. In this way, the actual elements making up the cassette for carrying out the normalization of the values on a known common scale are utilized.

Preferably, the cassette 11 has a different color than the test strip 13 which causes the luminous intensity of the light reflected, or emitted, by the cassette 11 outside the window 12 to be different from the luminous intensity of the light reflected, or emitted, by the test strip 13 outside the reactive zone RZ. For example, the test strip 13 is made of nitrocellulose and has a white color different from the color of the cassette 11, which causes the light reflected by the test strip 13 and the cassette 11 to be different and said information may be used to normalize the output signal S.

Even more preferably, the cassette 11 has a black color and the test strip 13 a white color. In this way, the difference between the light reflected, or emitted, by both elements is maximized. The white color of the test strip 13 absorbs virtually none of the light sent by the emitter 23, whereas the black color of the cassette 11 absorbs virtually all the light. In other words, the white color of the test strip 13 reflects virtually all the light, whereas the black color of the cassette 11 reflects virtually no light.

In the fourth step 304, luminous intensity variations in the output signal S of the sensor 24 are identified, and each of said variations may be a peak corresponding with a test line T or a control line C.

In the fifth step 305, after identifying the peaks in the output signal S the luminous intensity of the peaks of the test line T may be quantified, with the luminous intensity being related to the concentration of an analyte which is present in the test line T. For example, data may be stored in a memory with previous tests measured with the reading method of the invention and in which the luminous intensity values measured with the sensor 24 correlate to the concentration of the analyte in the test line T.

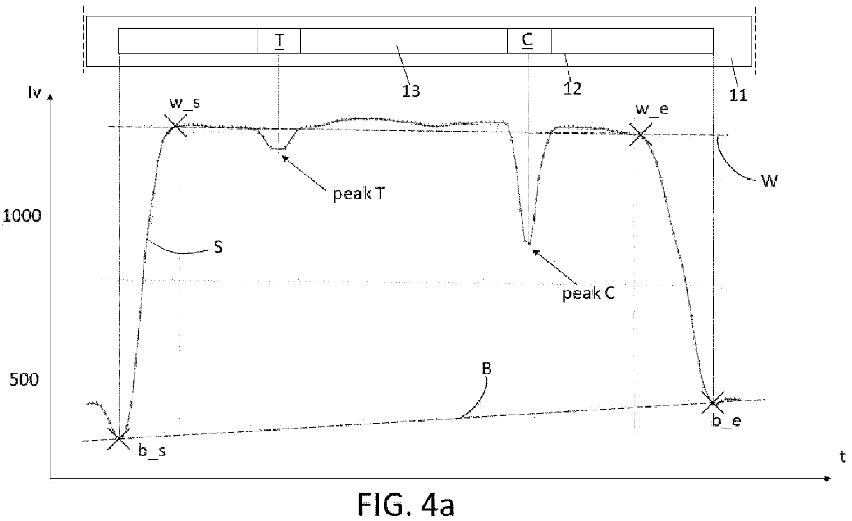

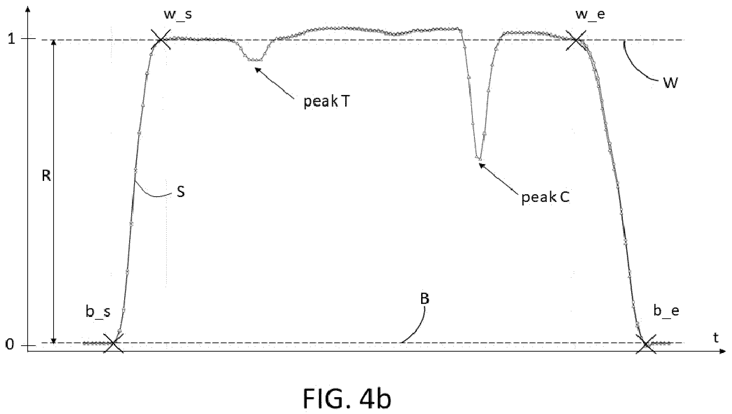

The sensor 24 may be a photodiode. Figure 4a shows raw data of the output signal S of a sensor in a single wavelength, which may have been obtained with a photodiode, and Figure 4b shows said signal after normalization.

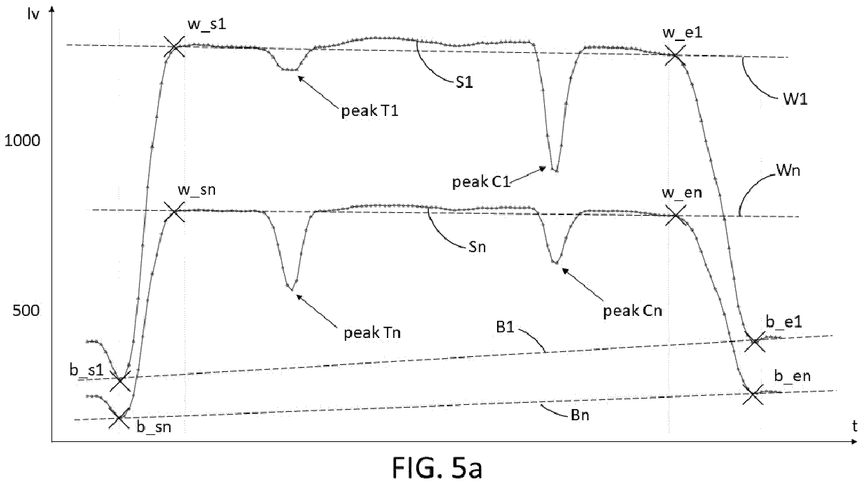

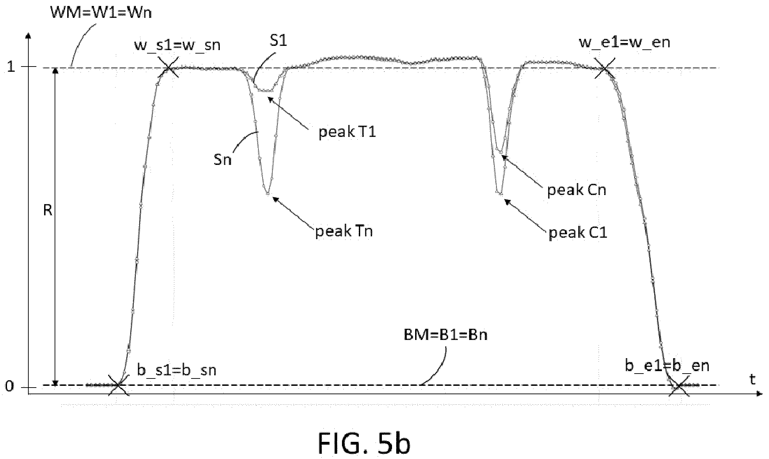

Preferably, the sensor 24 is a spectrometric sensor which obtains an output signal in channels, and each channel corresponds with the light reflected, or emitted, by the cassette in a certain wavelength. Figure 5a shows raw data of the output signal S of a spectrometric sensor in several wavelengths and Figure 5b shows the signal S of Figure 5a after normalization. The spectrometric sensor has several channels, for example 8, but for the sake of clarity only two channels S1 and Sn of the output signal S are represented.

In Figures 4a and 5a, the Y axis is represented in luminous intensity values "Iv", and in Figure 4b and 5b, the Y axis is adimensional, whereas the X axis is represented in all the figures in values of time "t".

As shown in Figure 4a and 4b, in the normalization of the output signal S a lower curve B and an upper curve W are obtained, and the range R is defined between the lower curve B and the upper curve W.

Figure 4a shows a correspondence between the raw data of the output signal S of the sensor and the cassette 11 that has the test strip 13. At least four points b_s, w_s, b_e and w_e are identified in said output signal S. The lower curve B is defined with at least one first point b_s and one second point b_e. The first point b_s corresponds with the light reflected, or emitted, by the cassette 11 at the start of the window 12 and the second point b_e corresponds with the light reflected, or emitted, by the cassette 11 at the end of the window 12. The upper curve W is defined with at least one third point w_s and one fourth point w_e. The third point w_s corresponds with the light reflected, or emitted, by the test strip 13 at the start of the window 12 and the fourth point w_e corresponds with the light reflected, or emitted, by the test strip at the end of the window 12. The points are identified by solving an optimization problem. The start and the end of the window 12 are the transitions existing between the cassette 11 and the test strip 13.

As observed in Figure 4b, the range R is defined between the lower curve B and the upper curve W. The lower curve B and upper curve W are two straight lines. The value '0' may be assigned to the lower curve and the value '1' may be assigned to the upper curve W. In the normalization, the raw data of the output signal S of the sensor is established proportionally between said curves lower B and upper W.

As shown in Figure 5a, when a spectrometric sensor is used the output signal S is represented in several channels S1 to Sn, and each channel corresponds with a wavelength. Said wavelengths may correspond with the wavelengths of the colors in the visible spectrum. For the sake of clarity, Figures 5a and 5b show only two channels S1 and Sn.

A lower curve B1 and Bn and an upper curve W1 and Wn are obtained for each channel S1 and Sn. Each curve is obtained in the same way as described in Figure 4a. The lower curve B1 is defined with at least one first point b_s1 and one second point b_e1. The lower curve Bn is defined with at least one first point b_sn and one second point b_en. The upper curve W1 is defined with at least one third point w_s1 and one fourth point w_e1. The upper curve Wn is defined with at least one third point w_sn and one fourth point w_en.

As shown in Figure 5b, in the normalization, the lower curves B1 and Bn of the channels S1 and Sn are superimposed on a main lower curve BM, and the upper curves W1 and Wn of the channels S1 and Sn are superimposed on a main upper curve WM, with the range R being defined between the main lower curve BM and the main upper curve WM, and with the luminous intensity values of the light of each channel S1 and Sn being established proportionally between the main lower curve BM and the main upper curve WM.

As is also observed in Figure 5b, the curves are straight lines. The value '0' may be assigned to the main lower curve BM and the value '1' may be assigned to the main upper curve WM. In the normalization, the raw data of all the channels S1 and Sn of the output signal S of the sensor 24 is established proportionally between said curves BM and WM.

Luminous intensity variations between the channels S1 and Sn are identified for each instant in time of the output signal S of the sensor 24, and each of said variations is a peak corresponding with a test line T or a control line C. As observed in Figure 5b, the channels S1 and Sn of the output signal S are superimposed at all the points except in the reactive zone RZ corresponding to the peaks T1, Tn, C1, and Cn of the test line T and control line C. It can be seen that the light reflected, or emitted, by the test line T and control line C show luminous intensity values different in each channel of the spectrometric sensor, whereas the light reflected, or emitted, by the test strip 13 outside the reactive zone RZ, or the light reflected, or emitted, by the cassette 11 outside the window, shows the same luminous intensity values in all the channels. In this way, luminous intensity variations that do not correspond to a test or control lines, such as defects in the test strip 13, for example, can be discriminated.

In Figures 4 and 5, the output signal S corresponds with a reflectance test, in which the peaks corresponding to the test line T and control line C are negative (in the form of valleys) since less light reaches the sensor due to the absorption of the particles used in the test line T and control line C. In a fluorescence test signal, the peaks would be positive, since the lines have fluorescent particles that absorb and then emit light.

## Claims

1. Reading method of a lateral flow test comprising:
- using a cassette (11) that has a window (12) through which a test strip (13) arranged in the cassette (11) is observed, the test strip (13) having a reactive zone (RZ) with at least one test line (T) and one control line (C),
- using a reader (20) that has a housing (21) for receiving the cassette (11), the reader (20) having an optical unit (22) with a light emitter (23) for illuminating the cassette (11) and a sensor (24) for receiving the light reflected, or emitted, by the cassette (11),
- manually moving the cassette (11) in the housing (21) of the reader (20),
- obtaining with the sensor (24) an output signal (S) comprising luminous intensity values of the light reflected, or emitted, by the cassette (11) during the time that the cassette (11) moves,
- normalizing the luminous intensity values of the output signal (S) of the sensor (24) in a range (R) defined between a lower value and a higher value, and
- identifying peaks in the output signal (S) of the sensor (24), said peaks corresponding with the test line (T) and the control line (C),
**characterized in that** the lower value and the higher value are established between the light reflected, or emitted, by the cassette (11) outside the window (12), and the light reflected, or emitted, by the test strip (13) outside the reactive zone (RZ), wherein the lower value corresponds with the light reflected, or emitted, by the cassette (11) outside the window (12), and the higher value corresponds with the light reflected, or emitted, by the test strip (13) outside the reactive zone (RZ), and wherein the cassette (11) has a different color than the test strip (13) which causes the luminous intensity of the light reflected, or emitted, by the cassette (11) outside the window (12) to be different from the luminous intensity of the light reflected, or emitted, by the test strip (13) outside the reactive zone (RZ).

2. Method according to claim 1, wherein the cassette (11) has a black color and the test strip (13) has a white color.

3. Method according to claim 1 or 2, wherein the light emitter (23) emits visible light on the cassette (11) and the sensor (24) receives the light directly reflected by the cassette (11).

4. Method according to any of claims 1 to 2, wherein the light emitter (23) emits UV light which is absorbed by the cassette (11) and the sensor (24) receives visible light emitted by the cassette (11), as a contrast to the UV light.

5. Method according to any of the preceding claims, wherein in the normalization of the output signal (S) a lower curve (B) and an upper curve (W) are obtained and the range (R) is defined between the lower curve (B) and the upper curve (W), the lower curve (B) is defined with at least one first point (b_s) and one second point (b_e), the first point (b_s) corresponding with the light reflected, or emitted, by the cassette (11) at the start of the window (12) and the second point (b_e) corresponding with the light reflected, or emitted, by the cassette (11) at the end of the window (12), and the upper curve (W) is defined with at least one third point (w_s) and one fourth point (w_e), the third point(w_s) corresponding with the light reflected, or emitted, by the test strip (13) at the start of the window (12) and the fourth point (w_e) corresponding with the light reflected, or emitted, by the test strip at the end of the window (12).

6. Method according to any of the preceding claims, wherein luminous intensity variations in the output signal (S) of the sensor (24) are identified, and each of said variations is a peak corresponding with a test line (T) or a control line (C).

7. Method according to any of the preceding claims, wherein after identifying the peaks in the output signal (S) the luminous intensity of the peaks of the test line (T) is quantified, with the luminous intensity being related to the concentration of an analyte which is present in the test line (T).

8. Method according to any of the preceding claims, wherein the sensor (24) is a photodiode.

9. Method according to any of claims 1 to 7, wherein the sensor (24) is a spectrometric sensor which obtains an output signal (S) in channels (S1,Sn), and each channel (S1,Sn) corresponds with the light reflected, or emitted, by the cassette (11) in a certain wavelength.

10. Method according to the preceding claim, wherein a lower curve (B1,Bn) and an upper curve (W1,Wn) are obtained for each channel (S1,Sn), the lower curves (B1,Bn) of the channels (S1,Sn) are superimposed on a main lower curve (BM), and the upper curves (W1,Wn) of the channels (S1,Sn) are superimposed on a main upper curve (WM), with the range (R) being defined between the main lower curve (BM) and the main upper curve (WM), with the luminous intensity values of the light of each channel (S1,Sn) being established proportionally between the main lower curve (BM) and the main upper curve (WM).

11. Method according to claim 9 or 10, wherein for each instant in time of the output signal (S) of the sensor (24) luminous intensity variations between the channels (S1, Sn) are identified, and each of said variations is a peak corresponding with a test line (T) or a control line (C).

12. Method according to any of the preceding claims, wherein the cassette (11) is manually moved between a first position in which the window (12) of the cassette (11) is inside the housing (21) of the reader (20) and a second position in which the window (12) of the cassette (11) is outside the housing (21) of the reader (20).

13. Method according to the preceding claim, wherein the output signal (S) is obtained during the movement of the cassette (11) between the first and the second position, and/or the output signal (S) is obtained during the movement of the cassette (11) between the second and the first position.

## Patentansprüche

1. Leseverfahren eines Lateralflusstests, umfassend:
- Verwenden einer Kassette (11) mit einem Fenster (12), durch das ein in der Kassette (11) angeordneter Teststreifen (13) sichtbar ist, wobei der Teststreifen (13) einen Reaktionsbereich (RZ) mit mindestens einer Testlinie (T) und einer Kontrolllinie (C) aufweist,
- Verwenden eines Lesegeräts (20) mit einem Gehäuse (21) zur Aufnahme der Kassette (11), wobei das Lesegerät (20) eine optische Einheit (22) mit einem Lichtemitter (23) zur Beleuchtung der Kassette (11) und einen Sensor (24) zum Empfang des von der Kassette (11) reflektierten oder emittierten Lichts aufweist,
- manuelles Bewegen der Kassette (11) im Gehäuse (21) des Lesegeräts (20),
- Erfassen eines Ausgangssignals (S) mit dem Sensor (24), das Lichtstärkewerte des von der Kassette (11) während der Bewegungsdauer der Kassette (11) reflektierten oder emittierten Lichts umfasst,
- Normalisieren der Lichtstärkewerte des Ausgangssignals (S) des Sensors (24) auf einen zwischen einem unteren Wert und einem oberen Wert definierten Bereich (R), und
- Erkennen von Spitzen im Ausgangssignal (S) des Sensors (24), wobei die genannten Spitzen der Testlinie (T) und der Kontrolllinie (C) entsprechen,
**dadurch gekennzeichnet, dass** der untere Wert und der obere Wert durch das von der Kassette (11) außerhalb des Fensters (12) reflektierte oder emittierte Licht und das von dem Teststreifen (13) außerhalb des Reaktionsbereichs (RZ) reflektierte oder emittierte Licht definiert sind, wobei der untere Wert dem von der Kassette (11) außerhalb des Fensters (12) reflektierten oder emittierten Licht und der obere Wert dem von dem Teststreifen (13) außerhalb des Reaktionsbereichs (RZ) reflektierten oder emittierten Licht entspricht, wobei die Kassette (11) eine andere Farbe als der Teststreifen (13) hat, wodurch sich die Lichtstärke des von der Kassette (11) außerhalb des Fensters (12) reflektierten oder emittierten Lichts von der Lichtstärke des von dem Teststreifen (13) außerhalb des Reaktionsbereichs (RZ) reflektierten oder emittierten Lichts unterscheidet.

2. Verfahren nach Anspruch 1, wobei die Kassette (11) von schwarzer Farbe und der Teststreifen (13) von weißer Farbe ist.

3. Verfahren nach Anspruch 1 oder 2, wobei der Lichtemitter (23) sichtbares Licht auf die Kassette (11) abgibt und der Sensor (24) das von der Kassette (11) direkt reflektierte Licht empfängt.

4. Verfahren nach einem der Ansprüche 1 bis 2, wobei der Lichtemitter (23) UV-Licht abgibt, das von der Kassette (11) absorbiert wird, und der Sensor (24) das von der Kassette (11) abgegebene sichtbare Licht als Kontrast zum UV-Licht empfängt.

5. Verfahren nach einem der vorstehenden Ansprüche, wobei bei der Normalisierung des Ausgangssignals (S) eine untere Kurve (B) und eine obere Kurve (W) ermittelt werden und der Bereich (R) zwischen der unteren Kurve (B) und der oberen Kurve (W) definiert ist, wobei die untere Kurve (B) durch mindestens einen ersten Punkt (b_s) und einen zweiten Punkt (b_e) definiert ist, wobei der erste Punkt (b_s) dem von der Kassette (11) am Anfang des Fensters (12) reflektierten oder emittierten Licht entspricht und der zweite Punkt (b_e) dem von der Kassette am Ende des Fensters (12) reflektierten oder emittierten Licht entspricht, und die obere Kurve (W) durch mindestens einen dritten Punkt (w_s) und einen vierten Punkt (w_e) definiert ist, wobei der dritte Punkt (w_s) dem von dem Teststreifen (13) am Anfang des Fensters (12) reflektierten oder emittierten Licht entspricht und der vierte Punkt (w_e) dem von dem Teststreifen am Ende des Fensters (12) reflektierten oder emittierten Licht entspricht.

6. Verfahren nach einem der vorstehenden Ansprüche, wobei im Ausgangssignal (S) des Sensors (24) Lichtstärkeabweichungen erkannt werden und jede der genannten Abweichungen eine einer Testlinie (T) oder einer Kontrolllinie (C) entsprechende Spitze ist.

7. Verfahren nach einem der vorstehenden Ansprüche, wobei nach Erkennen der Spitzen im Ausgangssignal (S) die Lichtstärke der Spitzen der Testlinie (T) quantifiziert wird, wobei die Lichtstärke mit der Konzentration eines in der Testlinie (T) vorhandenen Analyten in Bezug steht.

8. Verfahren nach einem der vorstehenden Ansprüche, wobei der Sensor (24) eine Fotodiode ist.

9. Verfahren nach einem der Ansprüche 1 bis 7, wobei der Sensor (24) ein spektrometrischer Sensor ist, der ein Ausgangssignal (S) in verschiedenen Kanälen (S1, Sn) erfasst, und die einzelnen Kanäle (S1, Sn) dem von der Kassette (11) reflektierten oder emittierten Licht in einer bestimmten Wellenlänge entsprechen.

10. Verfahren nach vorstehendem Anspruch, wobei für jeden Kanal (S1, Sn) eine untere Kurve (B1, Bn) und eine obere Kurve (W1, Wn) ermittelt werden, wobei die unteren Kurven (B1, Bn) der Kanäle (S1, Sn) eine untere Hauptkurve (BM) überlagern und die oberen Kurven (W1, Wn) der Kanäle (S1, Sn) eine obere Hauptkurve (WM) überlagern, wobei der Bereich (R) zwischen der unteren Hauptkurve (BM) und der oberen Hauptkurve (WM) definiert ist, wobei die Lichtstärkewerte des Lichts der einzelnen Kanäle (S1, Sn) proportional zwischen der unteren Hauptkurve (BM) und der oberen Hauptkurve (WM) festgelegt sind.

11. Verfahren nach Anspruch 9 oder 10, wobei für jeden Zeitpunkt des Ausgangssignals (S) des Sensors (24) Lichtstärkeabweichungen zwischen den Kanälen (S1, Sn) erkannt werden, und jede der genannten Abweichungen eine einer Testlinie (T) oder einer Kontrolllinie (C) entsprechende Spitze ist.

12. Verfahren nach einem der vorstehenden Ansprüche, wobei die Kassette (11) von einer ersten Position, in der sich das Fenster (12) der Kassette (11) im Gehäuse (21) des Lesegeräts (20) befindet, manuell in eine zweite Position, in der sich das Fenster (12) der Kassette (11) außerhalb des Gehäuses (21) des Lesegeräts (20) befindet, bewegt wird.

13. Verfahren nach vorstehendem Anspruch, wobei das Ausgangssignal (S) während der Bewegung der Kassette (11) von der ersten in die zweite Position und/oder während der Bewegung der Kassette (11) von der zweiten in die erste Position erfasst wird.

## Revendications

1. Procédé de lecture d'un test immunologique à flux latéral comprenant :
- l'utilisation d'une cassette (11) munie d'une fenêtre (12) à travers laquelle est visible une bandelette de test (13) disposée dans la cassette (11), ladite bandelette (13) comportant une zone réactive (ZR) avec au moins une ligne de test (T) et une ligne de contrôle (C),
- l'utilisation d'un lecteur (20) comprenant un boîtier (21) destiné à recevoir la cassette (11), le lecteur (20) étant équipé d'une unité optique (22) comprenant un émetteur lumineux (23) destiné à illuminer la cassette (11) et un capteur (24) destiné à recevoir la lumière réfléchie, ou émise, par la cassette (11),
- le déplacement manuel de la cassette (11) dans le boîtier (21) du lecteur (20),
- l'obtention, à l'aide du capteur (24), d'un signal de sortie (S) comprenant des valeurs d'intensité lumineuse de la lumière réfléchie, ou émise, par la cassette (11) pendant le déplacement de celle-ci,
- la normalisation des valeurs d'intensité lumineuse du signal de sortie (S) du capteur (24) dans une plage (R) définie entre une valeur minimale et une valeur maximale, et
- l'identification de pics dans le signal de sortie (S) du capteur (24), lesdits pics correspondant à la ligne de test (T) et à la ligne de contrôle (C),
**caractérisé en ce que** la valeur minimale et la valeur maximale sont établies entre la lumière réfléchie, ou émise, par la cassette (11) en dehors de la fenêtre (12), et la lumière réfléchie, ou émise, par la bandelette de test (13) en dehors de la zone réactive (ZR), la valeur minimale correspondant à la lumière réfléchie, ou émise, par la cassette (11) en dehors de la fenêtre (12), et la valeur maximale correspondant à la lumière réfléchie, ou émise, par la bandelette de test (13) en dehors de la zone réactive (ZR), et **en ce que** la cassette (11) a une couleur différente de celle de la bandelette de test (13), ce qui entraîne une intensité lumineuse différente entre la lumière réfléchie, ou émise, par la cassette (11) en dehors de la fenêtre (12) et celle réfléchie, ou émise, par la bandelette de test (13) en dehors de la zone réactive (ZR).

2. Procédé selon la revendication 1, dans lequel la cassette (11) est de couleur noire et la bandelette de test (13) est de couleur blanche.

3. Procédé selon la revendication 1 ou 2, dans lequel l'émetteur lumineux (23) émet de la lumière visible sur la cassette (11) et le capteur (24) reçoit la lumière directement réfléchie par la cassette (11).

4. Procédé selon l'une quelconque des revendications 1 à 2, dans lequel l'émetteur lumineux (23) émet de la lumière UV, laquelle est absorbée par la cassette (11), et le capteur (24) reçoit la lumière visible émise par la cassette (11), en contraste avec la lumière UV.

5. Procédé selon l'une quelconque des revendications précédentes, dans lequel, lors de la normalisation du signal de sortie (S), une courbe inférieure (B) et une courbe supérieure (W) sont obtenues, et la plage (R) est définie entre la courbe inférieure (B) et la courbe supérieure (W), la courbe inférieure (B) étant définie à partir d'au moins un premier point (b_s) et un deuxième point (b_e), le premier point (b_s) correspondant à la lumière réfléchie, ou émise, par la cassette (11) au début de la fenêtre (12), et le deuxième point (b_e) correspondant à la lumière réfléchie, ou émise, par la cassette (11) à la fin de la fenêtre (12), et la courbe supérieure (W) étant définie à partir d'au moins un troisième point (w_s) et un quatrième point (w_e), le troisième point (w_s) correspondant à la lumière réfléchie, ou émise, par la bandelette de test (13) au début de la fenêtre (12), et le quatrième point (w_e) correspondant à la lumière réfléchie, ou émise, par la bandelette de test à la fin de la fenêtre (12).

6. Procédé selon l'une quelconque des revendications précédentes, dans lequel des variations d'intensité lumineuse dans le signal de sortie (S) du capteur (24) sont identifiées, chacune de ces variations étant un pic correspondant à une ligne de test (T) ou à une ligne de contrôle (C).

7. Procédé selon l'une quelconque des revendications précédentes, dans lequel, après identification des pics dans le signal de sortie (S), l'intensité lumineuse des pics de la ligne de test (T) est quantifiée, ladite intensité lumineuse étant liée à la concentration d'un analyte présent dans la ligne de test (T).

8. Procédé selon l'une quelconque des revendications précédentes, dans lequel le capteur (24) est une photodiode.

9. Procédé selon l'une quelconque des revendications 1 à 7, dans lequel le capteur (24) est un capteur spectrométrique qui fournit un signal de sortie (S) sur des canaux (S1, Sn), chaque canal (S1, Sn) correspondant à la lumière réfléchie, ou émise, par la cassette (11) à une certaine longueur d'onde.

10. Procédé selon la revendication précédente, dans lequel une courbe inférieure (B1, Bn) et une courbe supérieure (W1, Wn) sont obtenues pour chaque canal (S1, Sn), les courbes inférieures (B1, Bn) des canaux (S1, Sn) étant superposées sur une courbe inférieure principale (BM), et les courbes supérieures (W1, Wn) des canaux (S1, Sn) étant superposées sur une courbe supérieure principale (WM), la plage (R) étant définie entre la courbe inférieure principale (BM) et la courbe supérieure principale (WM), les valeurs d'intensité lumineuse de la lumière de chaque canal (S1, Sn) étant établies proportionnellement entre la courbe inférieure principale (BM) et la courbe supérieure principale (WM).

11. Procédé selon la revendication 9 ou 10, dans lequel, pour chaque instant du signal de sortie (S) du capteur (24), des variations d'intensité lumineuse entre les canaux (S1, Sn) sont identifiées, chacune de ces variations étant un pic correspondant à une ligne de test (T) ou à une ligne de contrôle (C).

12. Procédé selon l'une quelconque des revendications précédentes, dans lequel la cassette (11) est déplacée manuellement entre une première position, dans laquelle la fenêtre (12) de la cassette (11) est à l'intérieur du boîtier (21) du lecteur (20), et une deuxième position, dans laquelle la fenêtre (12) de la cassette (11) est à l'extérieur du boîtier (21) du lecteur (20).

13. Procédé selon la revendication précédente, dans lequel le signal de sortie (S) est obtenu pendant le déplacement de la cassette (11) entre la première et la deuxième position, et/ou le signal de sortie (S) est obtenu pendant le déplacement de la cassette (11) entre la deuxième et la première position.
